# EUROPEAN PATENT APPLICATION

(11) **EP 4 752 209 A1**
(43) Date of publication of application: **03.06.2026**
(21) Application number: 24845340.9
(22) Date of filing: 03.07.2024
(51) Int. Cl.: C12M 1/26, C12M 1/34, G01N 1/04

(54) **COLLECTION ARRANGEMENT MEMBER, COLLECTION ARRANGEMENT DEVICE, AND COLLECTION ARRANGEMENT METHOD**

(30) Priority: 25.07.2023 JP 2023120825
(71) Applicant: NTN Corporation, Osaka-shi, Osaka 530-0005 (JP)
(72) Inventor: TSUKAMOTO, Yoshinari, Shizuoka 438-8510 (JP)
(74) Representative: Bockhorni & Brüntjen Partnerschaft Patentanwälte mbB
(86) International application number: PCT/JP2024/024064
(87) International publication number: WO 2025/022958

(57) **Abstract**

A collecting pin (141) having a hollowed shape and capable of cutting out and collecting a material to be treated, a disposing pin (142) that comes into contact with the material in the collecting pin (141) and pushes the material to dispose the material outside the collecting pin (141), and a fixing holder (143) to which the collecting pin (141) is fixed, are comprised. The material to be treated is a gel sheet, which is a gel formed into a sheet and supporting a target to be collected.

## Description

### TECHNICAL FIELD

The present invention relates to a collecting and disposing member, a collecting and disposing apparatus, and a collecting and disposing method.

### BACKGROUND ART

In recent years, a technique of selecting a target cell and a cell population through an analysis using a single cell has been rapidly developed. As the technique of selecting a target cell, cell selection methods such as a limiting dilution method, a cell sorter, a microfluidic channel, and a colony pick-up method have been attracting attention.

As a technique of handling a small amount of cells, a printing technique for handling a small amount of droplets has been attracting attention. The printing technique includes an inkjet scheme, a dispenser scheme, and an application scheme.

However, it is sometimes preferable to collect any cell only in any quantity. From the viewpoint of appropriately collecting any cell only in any quantity, a technique of highly accurately extracting a further minute amount of substance than the printing technique described above is required. From this viewpoint, a method for cutting and collecting any cell from a biological specimen or a group of cells by using a collecting needle disclosed in WO2017/061387 (PTL 1), has been developed.

As a method for applying a liquid droplet in a small amount, Japanese Patent Laying-Open No. 2020-015016 (PTL 2) discloses a technique using an application needle having an outer circumference surrounded by an outer cylinder moved relative to the application needle to form the liquid droplet to protrude outward from the tip of the application needle. According to Japanese Patent Laying-Open No. 2020-015016, a liquid material can be steadily applied.

### CITATION LIST

### PATENT LITERATURE

PTL 1: WO2017/061387
PTL 2: Japanese Patent Laying-Open No. 2020-015016

### SUMMARY OF INVENTION

### TECHNICAL PROBLEM

According to WO2017/061387, silicone rubber and gel come into contact with a cell specimen. The cell specimen is cut and temporarily accommodated in the collecting needle. Subsequently, liquid is injected into the collecting needle and the cell specimen in the collecting needle is discharged out of the collecting needle by the pressure of the liquid. Note, however, that the silicone rubber and the gel are not fixedly supported to surround tissue and cell. For this reason, the cell specimen has a sample collapsing and scattering when the cell specimen is discharged out of the collecting needle. The sample may not be collected and disposed in position accurately.

Furthermore, according to Japanese Patent Laying-Open No. 2020-015016, the outer cylinder is connected to a spring. Therefore, it is difficult to keep fixing a position at which the outer cylinder slides if the outer cylinder receives an external vertical force. That is, Japanese Patent Laying-Open No. 2020-015016 discloses a mechanism in which it is difficult to cut out a sample as the outer cylinder may unintentionally move when the outer cylinder is pressed against the sample.

The present invention has been made in view of the problem described above. An object of the present disclosure is to provide a collecting and disposing member, a collecting and disposing apparatus and a collecting and disposing method capable of collecting and disposing a sample with high stability, reproducibility and positional accuracy.

### SOLUTION TO PROBLEM

According to the present disclosure, a collecting and disposing member comprises a collecting pin, a disposing pin, and a fixing holder. The collecting pin has a hollowed shape and can cut and collect a material to be treated. The collecting pin presses the material to cut the material, and the disposing pin can be inserted into the collecting pin to come into contact with the cut material in the collecting pin and push the cut material to dispose the cut material outside the collecting pin. The collecting pin is fixed to the fixing holder. The material to be treated is a gel sheet, which is a gel formed into a sheet and supporting a target to be collected.

According to the present disclosure, a collecting and disposing apparatus comprises the collecting and disposing member described above and a holding table that holds a material cut with a collecting pin to be treated.

According to the present disclosure, in a collecting and disposing method, the collecting and disposing member described above is initially prepared. A material to be treated is disposed under the collecting pin, and the collecting pin is moved downward to cause its tip to cut the material and collects the cut material in the collecting pin, and the disposing pin is moved downward to bring its tip into contact with and push the cut material in the collecting pin to dispose the cut material outside the collecting pin.

In the presently disclosed collecting and disposing method, a gel that supports a target to be collected is formed into a sheet on a film to provide a gel sheet. The gel sheet on the film is punctured with a hollow collecting pin to cut and collect a portion of the gel sheet in the collecting pin, and the portion of the gel sheet is pressed with the disposing pin and thus pushed out of and disposed outside the collecting pin. In the step of puncturing, the disposing pin passes through the collecting pin.

### ADVANTAGEOUS EFFECTS OF INVENTION

According to the present disclosure, a collecting and disposing member, a collecting and disposing apparatus and a collecting and disposing method capable of collecting and disposing a sample with high stability, reproducibility and positional accuracy can be provided.

### BRIEF DESCRIPTION OF DRAWINGS

Fig. 1 is a schematic front view of an interior of a collecting and disposing apparatus according to an embodiment.
Fig. 2 is a front view of a collecting and disposing mechanism constituting a collecting and disposing member of Fig. 1 as viewed in a negative Y direction.
Fig. 3 is a side view of the collecting and disposing mechanism of Fig. 2 as viewed in a negative X direction.
Fig. 4 is a front view of the entirety of the collecting and disposing member of Fig. 1 as viewed in the negative Y direction.
Fig. 5 is a side view of the collecting and disposing member of Fig. 4 as viewed in the negative X direction.
Fig. 6 is a front view of the collecting and disposing member of Fig. 4 in an operation in the step of cutting and collecting a material to be treated, as viewed in the negative Y direction.
Fig. 7 is a side view in the operation of Fig. 6 as viewed in the negative X direction.
Fig. 8 is a front view of the collecting and disposing mechanism of Fig. 2 in an operation in a first step of ejecting and disposing the material to be treated, as viewed in the negative Y direction.
Fig. 9 is the front view in an operation in a second step following Fig. 8, as viewed in the negative Y direction.
Fig. 10 is the front view in an operation in a third step following Fig. 9, as viewed in the negative Y direction.
Fig. 11 is a side view in the operation of Fig. 10, as viewed in the negative X direction.
Fig. 12 is the front view in an operation in a fourth step following Fig. 10, as viewed in the negative Y direction.
Fig. 13 is the front view in an operation in a fifth step following Fig. 12, as viewed in the negative Y direction.
Fig. 14 is a schematic cross section for illustrating a first step of a collecting and disposing method according to the present embodiment.
Fig. 15 is a schematic cross section for illustrating a second step of the collecting and disposing method according to the present embodiment.
Fig. 16 is a schematic cross section for illustrating a third step of the collecting and disposing method according to the present embodiment.
Fig. 17 is a schematic cross section for illustrating a fourth step of the collecting and disposing method according to the present embodiment.
Fig. 18 is a schematic cross section for illustrating a fifth step of the collecting and disposing method according to the present embodiment.
Fig. 19 is a schematic enlarged perspective view of a plate and a gel plate on the plate as included in Fig. 18.
Fig. 20 is a schematic cross section for illustrating a sixth step of the collecting and disposing method according to the present embodiment.
Fig. 21 is a schematic enlarged cross section of a portion of the gel plate cut with the collecting pin immediately after Fig. 20.
Fig. 22 is a schematic enlarged cross section for illustrating a seventh step of the collecting and disposing method according to the present embodiment.
Fig. 23 is a schematic enlarged cross section for illustrating an eighth step of the collecting and disposing method according to the present embodiment.
Fig. 24 is a schematic cross section for illustrating an example of a step of dissolving a gel from a portion of a gel sheet to recover a cell alone.
Fig. 25 is a schematic enlarged cross section in which the sample in FIG. 22 is replaced with a factor.
Fig. 26 is a schematic enlarged cross section for illustrating a step of slowly releasing or eluting from the portion of the gel sheet shown in Fig. 25 a factor contained in the gel.
Fig. 27 is a schematic enlarged cross section for illustrating a first step of supplying a high viscosity solution into a container according to an exemplary variation.
Fig. 28 is a schematic enlarged cross section for illustrating a second step of supplying the high viscosity solution into the container according to the exemplary variation.
Fig. 29 is a schematic enlarged cross section for illustrating a third step of supplying the high viscosity solution into the container according to the exemplary variation.
Fig. 30 is a schematic enlarged cross section corresponding to Fig. 21 in an exemplary variation in which a high viscosity solution is supplied.
Fig. 31 is a schematic enlarged cross section corresponding to Fig. 22 in the exemplary variation in which the high viscosity solution is supplied.
Fig. 32 is a schematic enlarged cross section corresponding to Fig. 23 in the exemplary variation in which the high viscosity solution is supplied.
Fig. 33 is a photograph of a portion of a gel sheet that is collected with a first collecting pin.
Fig. 34 is a photograph of a portion of a gel sheet that is collected with a second collecting pin.
Fig. 35 is a photograph of a portion collected from a first gel sheet.
Fig. 36 is a photograph of a portion collected from a second gel sheet.

### DESCRIPTION OF EMBODIMENTS

Reference will now be made to the figures to describe an embodiment.

### (Collecting and Disposing Member)

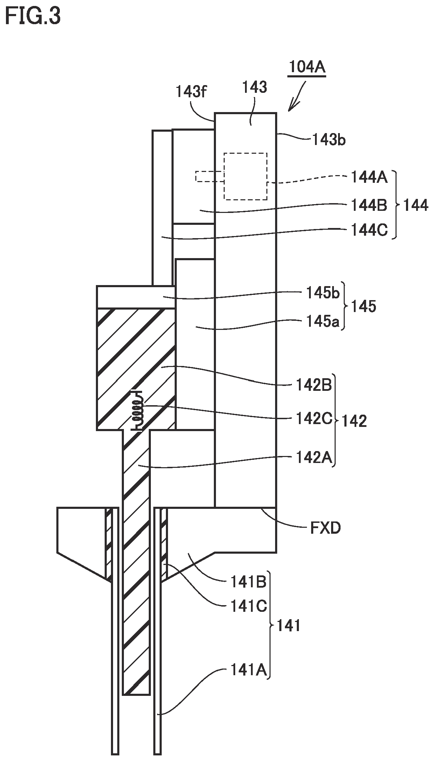

Initially, features of the present embodiment will briefly be described. Referring to Fig. 3, a collecting and disposing member (a collecting and disposing mechanism 104A) of the present embodiment comprises a collecting pin 141, a disposing pin 142, and a fixing holder 143. Collecting pin 141 has a hollowed shape and can cut out and collect a material to be treated. Collecting pin 141 is pressed against the material to cut the material, and disposing pin 142 can be inserted into collecting pin 141 to push the cut material out of collecting pin 141 and dispose the cut material at a desired position. Collecting pin 141 is fixed to fixing holder 143.

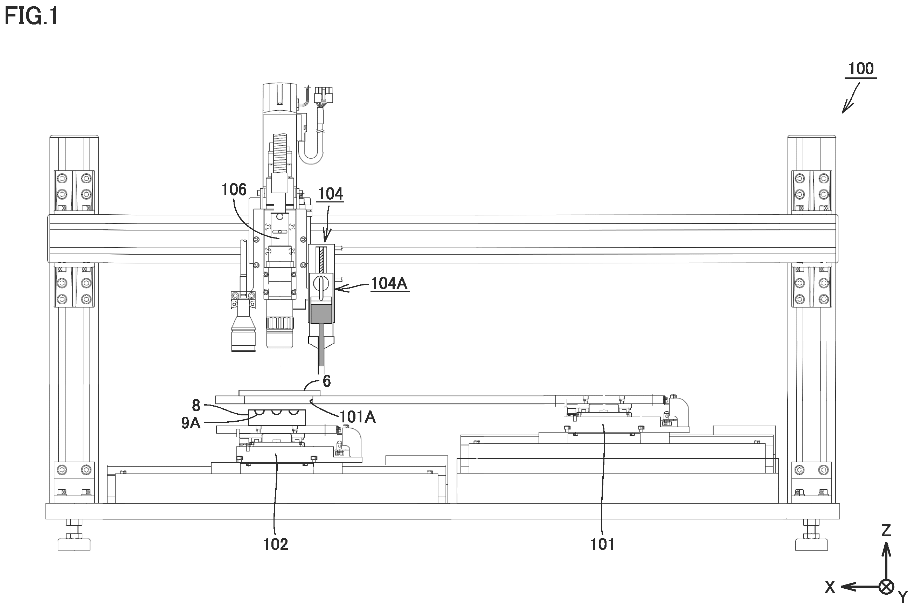

Fig. 1 is a schematic front view of an interior of a collecting and disposing apparatus according to the present embodiment. Note that, for convenience of description, an X direction, a Y direction, and a Z direction are introduced. Referring to Fig. 1, a collecting and disposing apparatus 100 is an apparatus for collecting a sample from a gel plate 6 serving as a sample described hereinafter. Collecting and disposing apparatus 100 comprises a treatment chamber, and an XY sample stage 101, an XY container stage 102 and a collecting and disposing member 104 disposed in the treatment chamber as major components. Note that while collecting and disposing apparatus 100 shown in Fig. 1 only includes a single collecting and disposing member 104, the apparatus may include a plurality of such collecting and disposing members.

XY sample stage 101 is movable in the horizontal direction, that is, the XY direction (the X direction and the Y direction). Specifically, for example, a guide section is provided on a lower surface of XY sample stage 101. The guide section is slidably connected to a guide rail provided on a bottom surface of the treatment chamber. XY sample stage 101 (a holding table) has an upper portion with a surface allowing gel plate 6 to be mounted thereon and fixed thereto. A stage penetrating section 101A is formed at at least a portion of XY sample stage 101 to penetrate XY sample stage 101. Gel plate 6 is preferably fixed to a position overlapping stage penetrating section 101A.

XY container stage 102 is disposed below XY sample stage 101. XY container stage 102 is movable in the horizontal direction, that is, the XY direction (the X direction and the Y direction). Specifically, for example, a guide section is provided on a lower surface of XY container stage 102. The guide section is slidably connected to a guide rail provided on a bottom surface of the treatment chamber. XY container stage 102 fixes a plate 8 in which a container 9A is formed to be capable of accommodating a portion of gel plate 6. Accordingly, in Fig. 1, collecting and disposing member 104, gel plate 6, and plate 8 in which container 9A is formed are disposed in this order from an upper side to a lower side in the Z direction.

Collecting and disposing member 104 and an optical observation system 106 are connected for example to a Z-axis table that is a member movable in the Z direction. That is, collecting and disposing member 104 and optical observation system 106 are held to be movable in the Z direction in collecting and disposing apparatus 100. Optical observation system 106 observes and measures a position and the like of a sample included in gel plate 6 to be collected. In optical observation system 106, a CCD camera that converts an observed image into an electrical signal may be installed. The observation of gel plate 6 and the like by optical observation system 106 may be performed with visible light. However, the observation of gel plate 6 and the like may be performed not only with visible light but also an infrared ray, an X ray, ultrasonically, or the like, and the observation of gel plate 6 is possible through magnetism depending on the material of gel plate 6. Gel plate 6 observed by a means other than visible light does not need to be transparent or semitransparent and may be nontransparent.

Although not shown, a control unit is installed outside the treatment chamber. The control unit includes a monitor, a computer for control, and a console panel. In the control unit, a command value for a speed of an operation of a needle for collection is input from the console panel and stored in a storage device of the computer for control. For example, when an operation is performed to collect a sample, the command value for the speed is read from the storage device and sent to a program for controlling collecting and disposing member 104. The program for controlling collecting and disposing member 104 determines a speed based on the command value to rotate a motor that is included in collecting and disposing member 104 to move collecting pin 141 and disposing pin 142 up and down at a predetermined speed. Collecting and disposing member 104 thus performs a collecting operation or the like. When the computer for control is communicating with a control system upstream thereof, the command value may be received from that control system. A parameter depending on material of a gel sheet from which a sample is collected may be stored in the storage device and a command value in speed for a collecting operation may be calculated according to a material of the gel sheet, a thickness of the gel sheet, and a collecting method, as designated.

### (Configuration of Collecting and Disposing Mechanism)

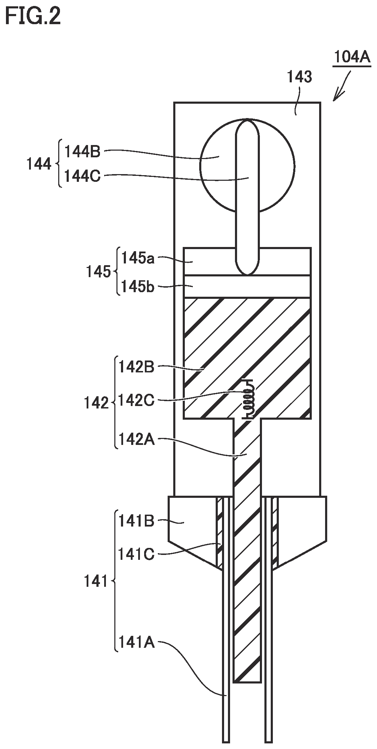

Fig. 2 is a front view of the collecting and disposing mechanism constituting the collecting and disposing member of Fig. 1 as viewed in a negative Y direction. Fig. 3 is a side view of the collecting and disposing mechanism of Fig. 2 as viewed in a negative X direction. Referring to Figs. 2 and 3, collecting and disposing member 104 of Fig. 1 includes a collecting and disposing mechanism 104A that is part of it. Collecting and disposing mechanism 104A comprises collecting pin 141, disposing pin 142, and fixing holder 143 as major components. Collecting pin 141 is a pin (a needle-shaped member) for cutting and collecting a material including a sample or the like to be treated. Disposing pin 142 is a pin for ejecting a sample collected and held by collecting pin 141, and disposing the collected and held sample at a desired position. Collecting pin 141 and disposing pin 142 are made of metal such as stainless steel or plastic.

Collecting pin 141 has a collecting pin body 141A and a collecting pin holder 141B. Collecting pin body 141A is in the form of a pin (or a pipe) and has a cylindrical shape hollowed at a center thereof in a cross section transverse to a direction in which the collecting pin body extends (or an upward/downward direction in Figs. 2 and 3, i.e., the Z direction). Collecting pin holder 141B is fitted on an outer circumferential surface of an upper portion of collecting pin body 141A. Therefore, collecting pin holder 141B is also hollow at a center thereof in a cross section transverse to the Z direction in Figs. 2 and 3. Collecting pin holder 141B is press-fitted and thus fixed to the outer circumferential surface of collecting pin body 141A. Alternatively, collecting pin holder 141B is adhered to the outer circumferential surface of collecting pin body 141A with an adhesive 141C. As shown in Figs. 2 and 3, collecting pin holder 141B may for example have a topmost surface in the XY plane whereas the collecting pin holder may have a bottommost surface inclined relative to the XY plane and thus have an outer circumferential side and an inner circumferential side such that the outer circumferential side becomes smaller in dimension in the Z direction than the inner circumferential side. Thus, collecting pin 141 generally has a hollowed shape.

Disposing pin 142 has a disposing pin body 142A and a disposing pin holder 142B. Disposing pin body 142A is in the form of a pin extending in the Z direction. Disposing pin body 142A may internally be solid, for example. Note that disposing pin body 142A is only required to at least have a bottommost surface in the Z direction with a center or the like closed without a hole, and may have a cavity inside. Disposing pin holder 142B is larger in dimension in the X direction (and the Y direction) than disposing pin body 142A. Disposing pin holder 142B holds disposing pin body 142A. For example, disposing pin holder 142B and disposing pin body 142A may have a bottom surface and a top surface, respectively, connected together so that they may be integrated together.

Disposing pin body 142A is dimensionally smaller than the hollow section of collecting pin body 141A in cross section. Disposing pin body 142A can thus be inserted into collecting pin body 141A. In contrast, disposing pin holder 142B is dimensionally larger than the hollow section of collecting pin body 141A in cross section. Thus, disposing pin holder 142B cannot be inserted into collecting pin body 141A.

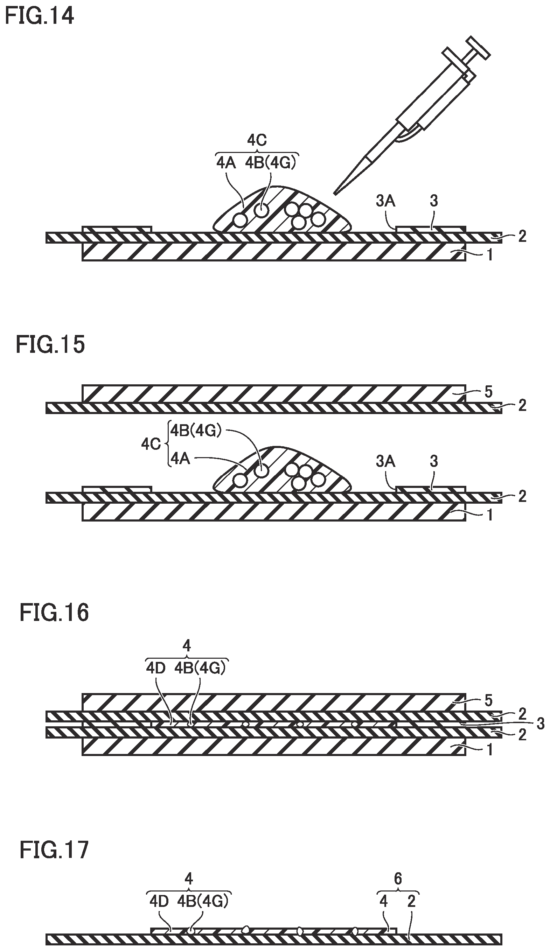

As collecting pin body 141A descends, the collecting pin body can press the material to be treated, cut out a portion of the material, and accommodate the cut-out portion inside the cylindrical hollow section. Note that the material to be treated is a gel sheet 4 in which a gel 4D that supports a target to be collected, or a sample 4B, is formed into a sheet (see Fig. 16 described hereinafter). Subsequently, disposing pin body 142A moves inside collecting pin body 141A. Disposing pin body 142A comes into contact with the material in collecting pin 141 and presses the material. Disposing pin body 142A thus pushes out of collecting pin 141 the material accommodated in collecting pin body 141A. Disposing pin body 142A can move in the hollow section of collecting pin body 141A in an upward/downward direction. That is, disposing pin body 142A passes through collecting pin body 141A. Disposing pin body 142A is moved in the upward/downward direction by a first drive unit 144 described hereinafter. The material to be treated that is cut out can thus be disposed at a desired position.

A spring 142C is provided in a region of disposing pin holder 142B adjacent to disposing pin body 142A, that is, a bottommost region of disposing pin holder 142B. Spring 142C connects disposing pin holder 142B to a topmost portion of disposing pin body 142A. Spring 142C absorbs a force exerted to disposing pin holder 142B in the Z direction when disposing pin body 142A collides with a material to be treated such as a substrate. Disposing pin 142 may not have spring 142C. When disposing pin 142 does not have spring 142C, the force exerted at the time of collision can no longer be absorbed. However, when disposing pin 142 does not have spring 142C, precisely controlling a position to which disposing pin 142 descends can eliminate an effect of the collision.

Fixing holder 143 can hold collecting pin 141. Fixing holder 143 is disposed on an upper side of collecting pin 141 and on a back side of disposing pin 142 with reference to Fig. 2. While fixing holder 143 is for example in the form of a plate (a rectangular parallelepiped), this is not exclusive. Collecting pin 141 is fixed to fixing holder 143. Specifically, collecting pin holder 141B and fixing holder 143 have a topmost surface and a bottommost surface, respectively, in contact with each other at and thus fixed together at a fixing portion FXD. They may be detachably attached and thus fixed together at fixing portion FXD for example by a stainless steel screw screwed in a vicinity of fixing portion FXD of collecting pin holder 141B and a magnetic force of a magnet embedded in a vicinity of fixing portion FXD of fixing holder 143. Alternatively, they may be detachably attached and thus fixed together at fixing portion FXD by screwing with fixing holder 143 a stainless steel screw screwed to collecting pin holder 141.

First drive unit 144 is provided on a front surface 143f of fixing holder 143 (a surface thereof in the negative Y direction) on a side upper in the Z direction than disposing pin holder 142B. First drive unit 144 includes a first motor 144A, a disk member 144B, and a link member 144C. First drive unit 144 is attached to a disposing pin holder fixing unit 145.

Disposing pin holder fixing unit 145 has a first portion 145a and a second portion 145b. First portion 145a is in the form of a plate disposed to have a back major surface in contact with front surface 143f of fixing holder 143 and thus be slidable on front surface 143f. Second portion 145b is a plate-shaped portion disposed on a front major surface of first portion 145a opposite to the major surface thereof in contact with front surface 143f so as to be substantially orthogonal to first portion 145a. Fixing holder 143 may have front surface 143f for example with a groove formed to extend in the Z direction to allow disposing pin holder fixing unit 145 to slide along the groove.

Link member 144C is disposed on a front side of disk member 144B with reference to Fig. 2. Link member 144C has an elongate shape extending in the upward/downward direction, that is, the Z direction. Disk member 144B has a disk-shaped back major surface in contact with front surface 143f of fixing holder 143. First motor 144A is attached to a position overlapping a round center portion of disk member 144B. First motor 144A may have at least a portion inside fixing holder 143. Disk member 144B is rotatable about its center by first motor 144A. Link member 144C in the direction in which it extends has one end, or a first end, fixed to a portion of an outer circumference of disk member 144B.

Link member 144C in the direction in which it extends (i.e., the Z direction) has a second end opposite to the first end (i.e., a lower end in Fig. 2) fixed to disposing pin holder fixing unit 145 in contact with disposing pin holder fixing unit 145 at first portion 145a. The second end may be contactable with disposing pin holder fixing unit 145 at second portion 145b. As first motor 144A rotates disk member 144B, link member 144C moves in the upward/downward direction and accordingly, the second end moves disposing pin 142 in the upward/downward direction. This will be described more specifically hereinafter.

In order to cause such an operation, disposing pin holder fixing unit 145 is provided between the second end and disposing pin 142 (or disposing pin holder 142B), and disposing pin 142 is fixed thereto. Disposing pin 142 is fixed in contact with disposing pin holder fixing unit 145 at both first portion 145a and second portion 145b. Disposing pin 142 (or disposing pin holder 142B) and disposing pin holder fixing unit 145 (or first portion 145a and second portion 145b) may be detachably attached and thus fixed together for example by a magnetic force of a magnet embedded in disposing pin holder 142B and a magnet embedded in first portion 145a, for example. Alternatively, they may be screwed together, and thus detachably attached and thus fixed together.

### (General Configuration of Collecting and Disposing Member)

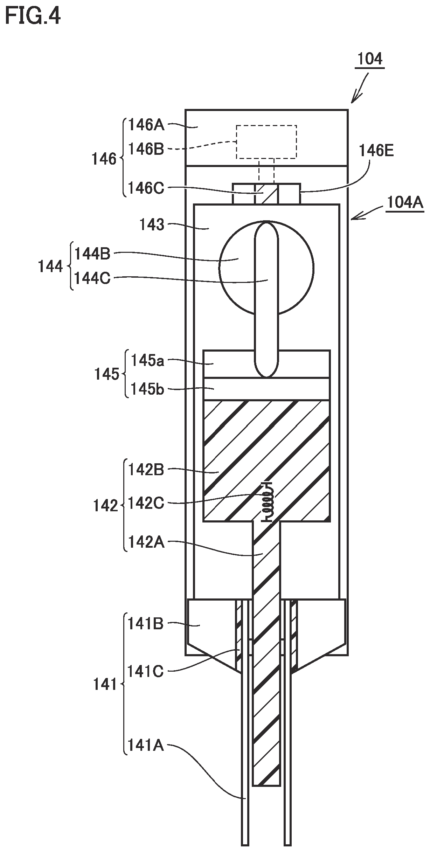

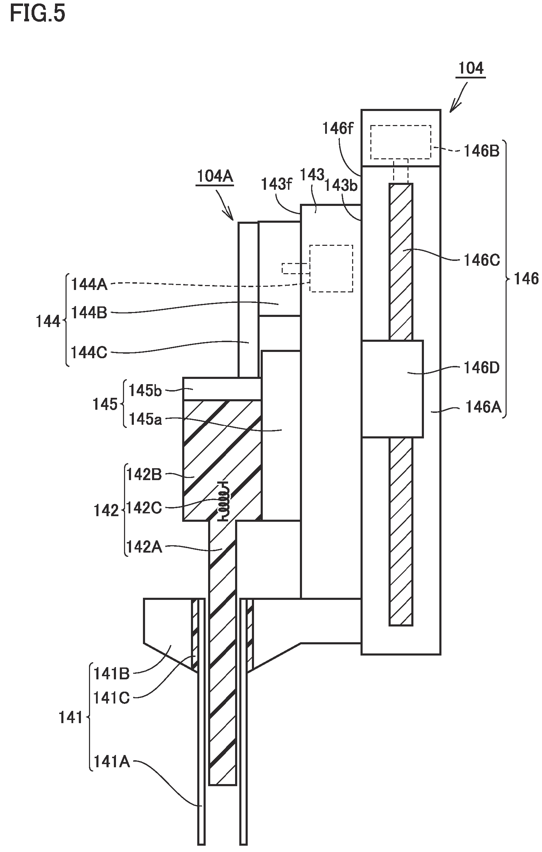

Fig. 4 is a front view of the entirety of the collecting and disposing member of Fig. 1 as viewed in the negative Y direction. Fig. 5 is a side view of the collecting and disposing member of Fig. 4 as viewed in the negative X direction. Referring to Figs. 4 and 5, collecting and disposing member 104 further comprises a second drive unit 146 in addition to collecting and disposing mechanism 104A of Figs. 2 and 3. Second drive unit 146 is disposed on a back surface of fixing holder 143 of Figs. 2 and 3 and includes a movable holder 146A. Movable holder 146A is for example in the form of a plate (a rectangular parallelepiped) and accommodates a member therein, although this is not exclusive. Movable holder 146A has a larger dimension in the Z direction than fixing holder 143. Collecting and disposing mechanism 104A is attached to movable holder 146A.

Second drive unit 146 is a slide mechanism causing collecting and disposing mechanism 104A including fixing holder 143 to slide in the Z direction. Second drive unit 146 includes other than movable holder 146A a second motor 146B, a vertically extending screw 146C, and a screw coupling unit 146D. Vertically extending screw 146C extends in the upward/downward direction and is externally threaded. Vertically extending screw 146C is disposed in movable holder 146A provided for example in the form of a rectangular parallelepiped, and has a larger dimension in the Z direction than fixing holder 143. Vertically extending screw 146C may be accommodated in a groove formed in a front surface 146f of movable holder 146A and extending in the Z direction. In movable holder 146A vertically extending screw 146C has one end (an upper end) with second motor 146B attached thereto. Screw coupling unit 146D is attached to fixing holder 143 on a back surface 143b (a surface opposite to front surface 143f) so that vertically extending screw 146C and fixing holder 143 are connectable together. Screw coupling unit 146D is internally threaded to allow the externally threaded, vertically extending screw 146C to be screwed and thus fastened thereto.

Vertically extending screw 146C is rotated by second motor 146B. While vertically extending screw 146C rotates, it does not move vertically, whereas screw coupling unit 146D fastened to vertically extending screw 146C moves vertically. This moves fixing holder 143 attached to screw coupling unit 146D and collecting and disposing mechanism 104A including collecting pin 141 as a whole vertically. This will be described in detail later.

### (Collecting Step)

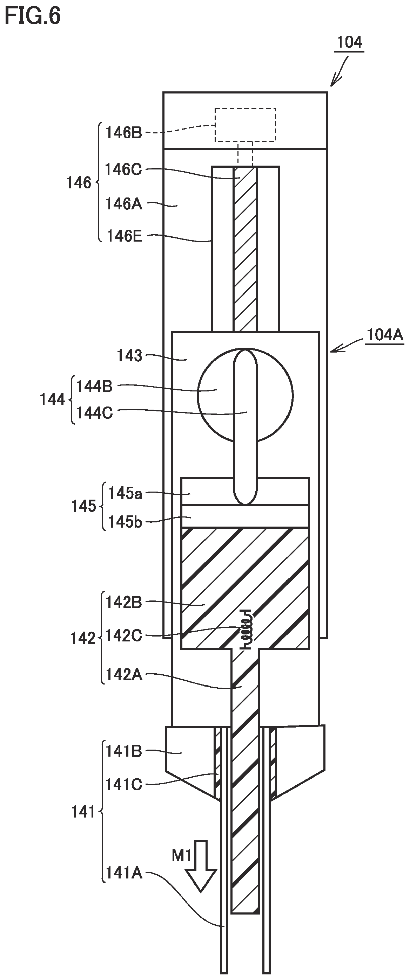

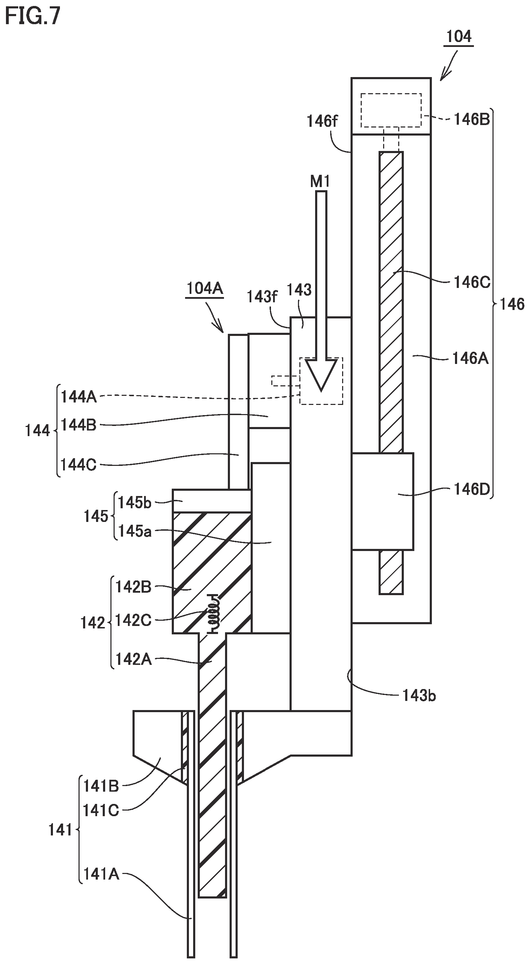

Fig. 6 is a front view of the collecting and disposing member of Fig. 4 in an operation in the step of cutting and collecting a material to be treated, as viewed in the negative Y direction. Fig. 7 is a side view in the operation of Fig. 6 as viewed in the negative X direction. Referring to Figs. 6 and 7, the rotational motion of second motor 146B is converted into the vertical motion of screw coupling unit 146D. Screw coupling unit 146D and back surface 143b of fixing holder 143 are screwed and thus fixed together. Therefore, as screw coupling unit 146D vertically moves, fixing holder 143 and collecting and disposing mechanism 104A including and collecting pin 141 move as a whole vertically. It is preferable that a slide groove 146E (see Fig. 6) extending in the Z direction be formed in front surface 146f of movable holder 146A facing back surface 143b of fixing holder 143 so that screw coupling unit 146D slides along the groove. Slide groove 146E is formed from front surface 146f in a positive Y direction depthwise (or dug from a front side with respect to the plane of the sheet of Fig. 6 depthwise). In this case, screw coupling unit 146D is disposed to be at least partially fit in slide groove 146E.

For example, collecting and disposing mechanism 104A moves downward as indicated in Figs. 6 and 7 by an arrow M1. This allows the tip of collecting pin 141 (collecting pin body 141A) to cut a portion of the material to be treated while the material is disposed below the tip. The portion cut out of the material to be treated is accommodated in the hollow section of collecting pin body 141A.

### (Disposing Step)

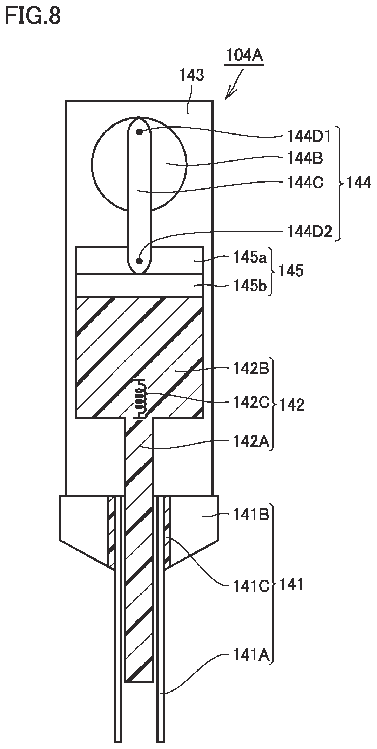

Fig. 8 is a front view of the collecting and disposing mechanism of Fig. 2 in an operation in a first step of ejecting and disposing the material to be treated, as viewed in the negative Y direction. Referring to Fig. 8, link member 144C has the first end fixed to a portion of an outer circumference of disk member 144B by a joint 144D1. Link member 144C has the second end fixed to disposing pin holder fixing unit 145 by a joint 144D2. Fig. 8 shows an initial state, in which link member 144C has the first end at a topmost portion of disk member 144B. Accordingly, disposing pin holder fixing unit 145 connected to link member 144C and disposing pin 142 are located in the Z direction at their respective topmost locations. Disposing pin body 142A has its tip (or bottommost portion) above the bottommost portion of collecting pin body 141A. In this state, the bottommost portion of collecting pin body 141A is the bottommost portion of the entirety, and this portion can be used to cut out the material to be treated.

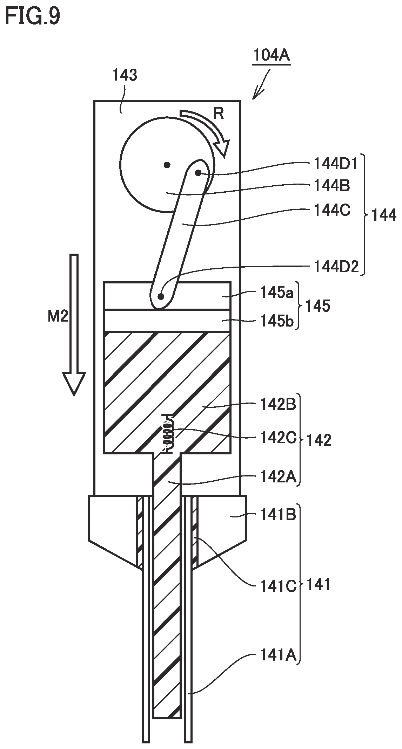

Fig. 9 is the front view in an operation in a second step following Fig. 8, as viewed in the negative Y direction. Referring to Fig. 9, first motor 144A (see Fig. 3) rotates disk member 144B in a direction indicated in the figure by an arrow R. This moves the first end of link member 144C to a center position of disk member 144B in the Z direction. Accordingly, while link member 144C is slightly inclined with respect to the Z direction, the link member moves disposing pin holder fixing unit 145 connected thereto and disposing pin 142 downward as indicated in the figure by an arrow M2.

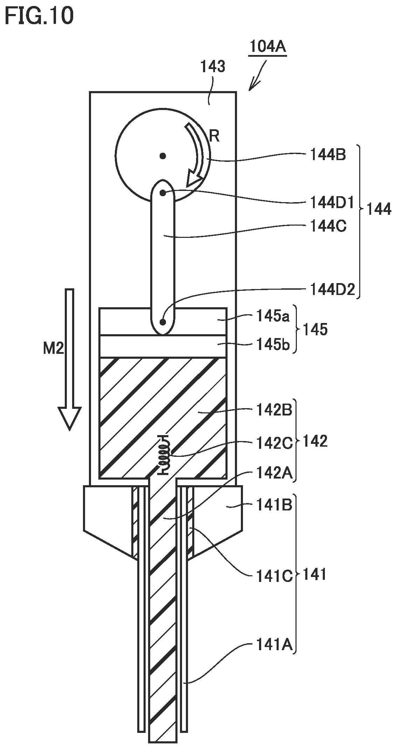

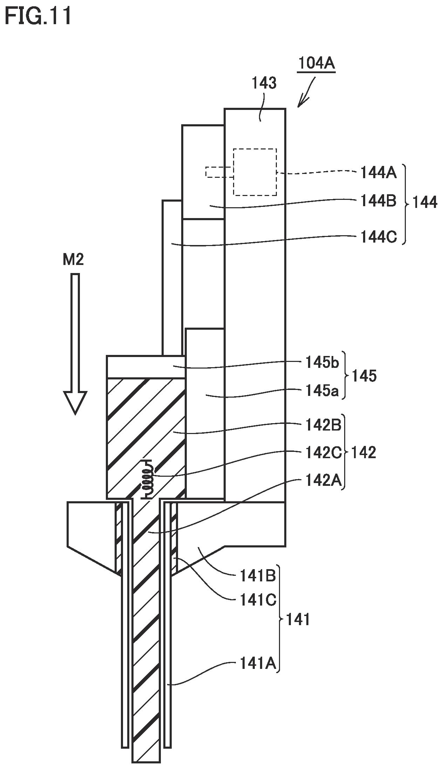

Fig. 10 is the front view in an operation in a third step following Fig. 9 as viewed in the negative Y direction. Fig. 11 is a side view in the operation of Fig. 10 as viewed in the negative X direction. Referring to Figs. 10 and 11, disk member 144B further rotates from the state of Fig. 9, and the first end reaches the bottommost portion of disk member 144B. When this is done, as indicated in the figure by arrow M2, disposing pin 142 moves downward to a side lowest relative to collecting pin 141, and as the downward movement is made, disposing pin holder fixing unit 145 and disposing pin 142 also descend to their respective lowest sides. When disposing pin 142 moves downward, the tip of disposing pin body 142A slides downward so as to pass through the hollow section of collecting pin body 141A. As a result, the tip of disposing pin body 142A comes into contact with the portion of the material to be treated that is accommodated inside collecting pin body 141A, presses the portion downward, and ejects the portion out of collecting pin body 141A to dispose the portion at a desired position.

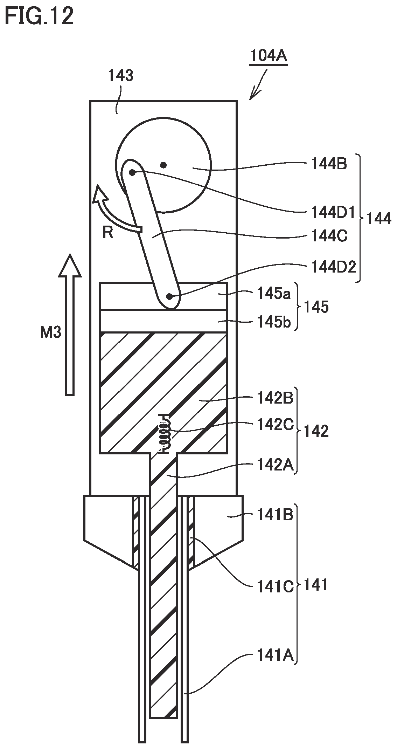

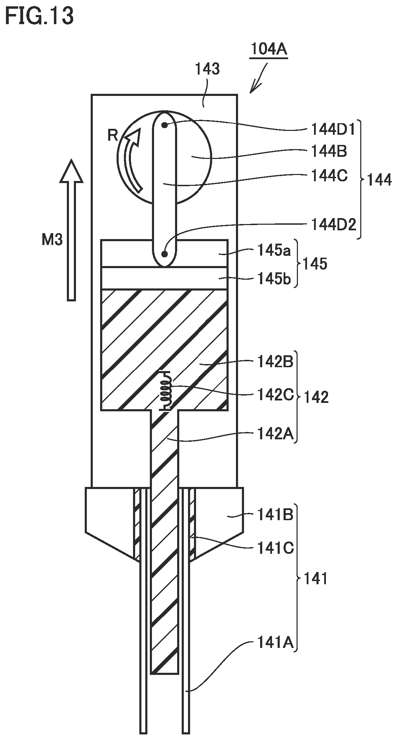

Fig. 12 is the front view in an operation in a fourth step following Fig. 10, as viewed in the negative Y direction. Fig. 13 is the front view in an operation in a fifth step following Fig. 12, as viewed in the negative Y direction. Referring to Figs. 12 and 13, when disk member 144B further rotates, disposing pin 142 and disposing pin holder fixing unit 145 are moved in a reverse direction, as indicated in the figures by an arrow M3, and return to the initial state of Fig. 8, as shown in Fig. 13.

An exemplary variation of joints 144D1 and 144D2 may be provided in the following manner. For example, it may be a mechanism in which there may be a spring connecting disposing pin holder fixing unit 145 and link member 144C together, and when link member 144C presses downward, disposing pin holder fixing unit 145 may descend; otherwise the spring's biasing force is applied so that disposing pin holder fixing unit 145 may ascend.

### (Details of Method for Collecting and Disposing a Target)

Fig. 14 is a schematic cross section for illustrating a first step of the collecting and disposing method according to the present embodiment. Referring to Fig. 14, film 2 is placed for example on slide glass 1. As an example, slide glass 1 is a thin plate material made of glass having a thickness of approximately 1.2 mm. Film 2 is formed of a soft material thinly to be easily torn by a needle puncturing the film. As an example, film 2 is, for example, polyvinylidene chloride having a thickness of approximately 10 µm. That is, as film 2, for example, a thin film for food packaging or a thin film of silicone rubber is preferably used. A spacer 3 is disposed on film 2. Spacer 3 may be formed of the same glass material as a glass material forming slide glass 1. Spacer 3 may be 100 µm or more and 200 µm or less in thickness. For example, spacer 3 may be 100 µm or may be 200 µm in thickness. Spacer 3 has a through hole 3A formed at a center of the spacer as viewed from an upper side of Fig. 14 (in plan view). Through hole 3A penetrates spacer 3. Accordingly, through hole 3A is internally hollow and has no material, such as glass that forms spacer 3, disposed therein. Through hole 3A in plan view has any shape such as a rectangular shape.

Subsequently, a fluid sample 4C having fluidity and composed of a gel material 4A having fluidity and capable of being gelatinized and one or more samples 4B included in the gel material as a target is supplied into through hole 3A. Sample 4B is a cell to be collected or the like. Fig. 14 shows a plurality of samples 4B disposed one by one such that they are spaced from one another. Gel material 4A is, for example, 1.5% agarose gel obtained by adding a fluorescent dye for example to 1.5 mL of a gel precursor. However, gel material 4A is not limited to this and may for example be collagen or may be a mixture of alginic acid and collagen.

Fig. 15 is a schematic cross section for illustrating a second step of the collecting and disposing method according to the present embodiment. Referring to Fig. 15, film 2 is stuck on a lower surface of slide glass 5. Slide glass 5 may be of the same material and size as slide glass 1 shown in Fig. 14. In Fig. 15, film 2 above fluid sample 4C may be of the same material and size as film 2 shown in Fig. 14.

Fig. 16 is a schematic cross section for illustrating a third step of the collecting and disposing method according to the present embodiment. Referring to Fig. 16, slide glass 5 with film 2 stuck thereon is placed on spacer 3. Film 2 stuck on slide glass 5 comes into contact with fluid sample 4C and spacer 3. Slide glass 5 and film 2 press fluid sample 4C and spacer 3 downward. Fluid sample 4C and spacer 3 are thus sandwiched by the underlying slide glass 1 with film 2 stuck thereon and the overlying slide glass 5 with film 2 stuck thereon. Thus, slide glass 1, film 2, spacer 3 and fluid sample 4C, film 2, and slide glass 5 are stacked in this order from the lower side to the upper side.

Subsequently, for example, when gel material 4A is a material that solidifies depending on temperature, the stack is entirely cooled to 4°C. As the stack is cooled, gel material 4A forming fluid sample 4C is solidified to be gel 4D, and gel sheet 4 having sample 4B supported inside gel 4D is formed in through hole 3A. Although solidified, gel 4D is soft enough to be easily torn by a needle puncturing the gel. Note that sample 4B being supported by gel 4D means, for example, that sample 4B is embedded such that it is surrounded by and covered with, and thus buried in hard gel 4D and sample 4B does not flow.

Fig. 17 is a schematic cross section for illustrating a fourth step of the collecting and disposing method according to the present embodiment. Referring to Fig. 17, after gel sheet 4 is provided, film 2 and slide glass 5 placed on the upper side of gel sheet 4 are removed and slide glass 1 and spacer 3 are also removed. Thus, gel plate 6 including film 2 and gel sheet 4 placed on film 2 is formed. In gel sheet 4, sample 4B to be collected is supported by the solidified gel 4D for example by being embedded therein. Gel 4D is formed into a sheet on film 2. That is, gel sheet 4 includes gel 4D formed into a sheet on film 2 and supporting sample 4B to be collected. In the present embodiment, "providing gel sheet 4" means that the exact step of embedding sample 4B in gel 4D or the like to support the sample to obtain gel sheet 4 may be performed on film 2, or gel sheet 4 for example externally, previously formed may be attached on film 2 later. A method for forming gel sheet 4 may be previously preparing it on any flat surface.

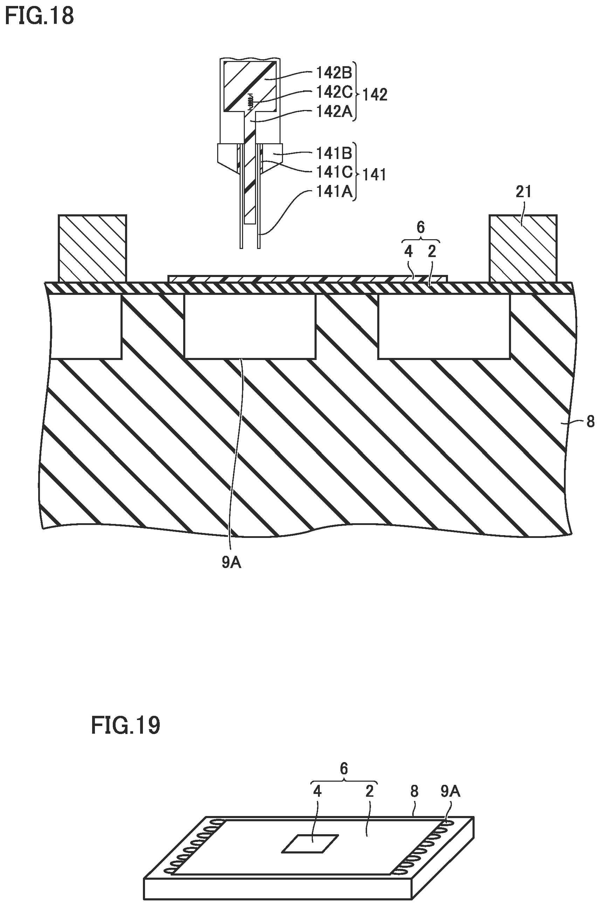

Fig. 18 is a schematic cross section for illustrating a fifth step of the collecting and disposing method according to the present embodiment. Fig. 19 is a schematic enlarged perspective view of a plate and a gel plate on the plate as included in Fig. 18. Referring to Figs. 18 and 19, gel plate 6 thus formed is punctured by collecting pin 141 as it descends from above. When the puncture is done, collecting pin 141 descends through a mechanism, as has been described above (see Figs. 6 and 7).

Gel plate 6 is placed on plate 8. Plate 8 is a culture plate for cell culture. A plurality of containers 9A serving as a recovering section are formed in plate 8. The plurality of containers 9A are recessed portions of plate 8 that are formed by recessing an upper surface of the plate. As container 9A has a recessed form, it can accommodate therein a portion of gel sheet 4 cut out (a portion 4E of the gel sheet described hereinafter). After the portion of gel sheet 4 (or portion 4E of the gel sheet described hereinafter) is cut out before it is disposed it may be pulled out of container 9A and film 2 while held in the needle (or collecting pin body 141A) and may be accommodated in a separately prepared container or the like.

The plurality of containers 9A may be spaced from one another for example in eight rows depthwise in Fig. 19 and 12 columns laterally in the figure for a total of 96 containers. Container 9A in plan view has any shape such as a round shape. In Figs. 18 and 19, film 2 forming gel plate 6 is in contact with an upper surface of plate 8 to close at least a portion of the plurality of containers 9A. To do so, plate 8 may have a portion (a region on an upper side) within stage penetrating section 101A shown in Fig. 1.

A sheet fixing member 21 may be placed above gel plate 6. Sheet fixing member 21 suppresses positional displacement of gel plate 6 caused by force applied to gel 4D from above when the step of cutting gel sheet 4 is performed. As a means for suppressing positional displacement of gel plate 6, an adhesive film tape may be used instead of sheet fixing member 21.

Plate 8 having container 9A formed therein as a culture container is formed of any material selected from the group consisting of polystyrene, polypropylene, polycarbonate, and polyethylene terephthalate.

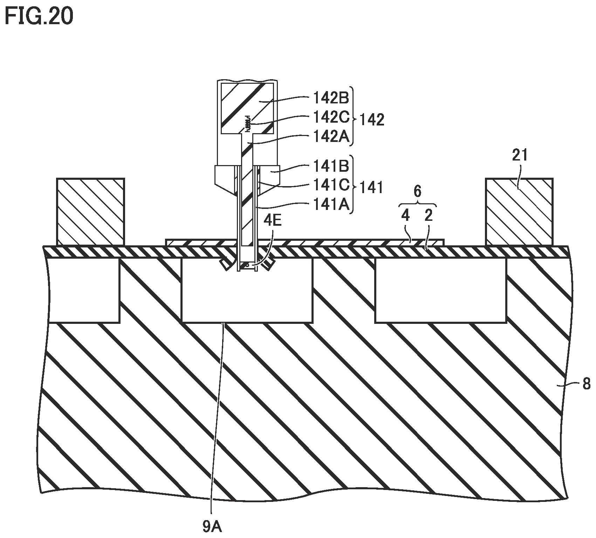

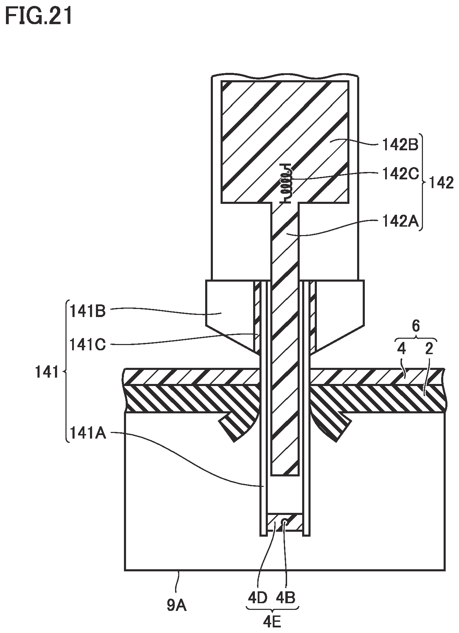

Fig. 20 is a schematic cross section for illustrating a sixth step of the collecting and disposing method according to the present embodiment. Fig. 21 is a schematic enlarged cross section of a portion of the gel plate cut out by the collecting pin immediately after Fig. 20. Referring to Figs. 20 and 21, collecting and disposing member 104 including collecting pin 141 entirely descends relative to the state shown in Fig. 18. Collecting pin 141 should be moved downward to a position where sample 4B as a target to be collected is present, as seen from an image obtained through an optical observation system (e.g., optical observation system 106 shown in Fig. 1). For example, gel plate 6 has gel sheet 4, in particular, punctured with collecting pin body 141A while, as shown in Fig. 8, disposing pin body 142A is located at its topmost position and collecting pin body 141A has its bottommost portion below the bottommost portion of disposing pin body 142A. When this is done, it is preferable that collecting pin body 141A at least have a hollow section (i.e., a hollow section between the bottommost portion of collecting pin body 141A and the bottommost portion of disposing pin body 142A) with a volume maintained to be a sufficient volume that can accommodate therein the portion cut out of the gel sheet.

The portion of gel sheet 4 punctured is torn as a sharp tip of collecting pin body 141A passes therethrough. Collecting pin body 141A penetrates gel sheet 4 while tearing gel sheet 4. A portion of gel sheet 4 cut to be separated from the original gel sheet 4 by collecting pin body 141A is a portion 4E of the gel sheet. Portion 4E of the gel sheet to be cut out is preferably composed of gel 4D and sample 4B embedded in the gel. This prevents sample 4B from flowing with respect to gel 4D. This can suppress collapse and scattering of sample 4B when portion 4E of the gel sheet is collected in collecting pin 141 and disposed. Portion 4E of the gel sheet cut out is collected (or accommodated) in collecting pin body 141A.

After collecting pin body 141A cuts portion 4E out of the gel sheet, collecting pin body 141A further descends while holding portion 4E of the gel sheet therein (or in its hollow section). Collecting pin body 141A thus penetrates gel plate 6 at a portion of film 2 located directly under gel sheet 4. Thus, film 2 is also torn as well as gel sheet 4.

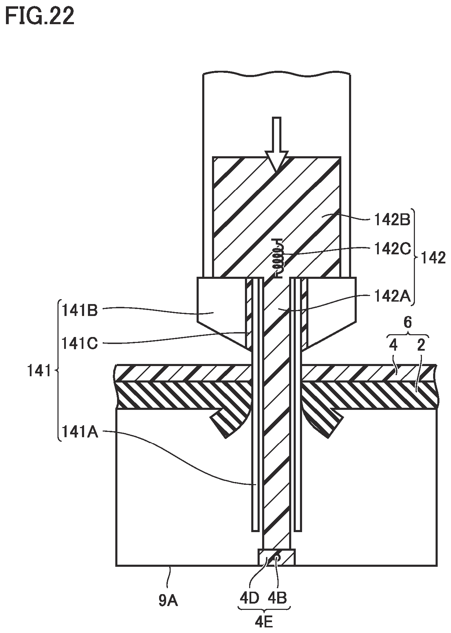

Fig. 22 is a schematic enlarged cross section for illustrating a seventh step of the collecting and disposing method according to the present embodiment. Referring to Fig. 22, after Fig. 21, disposing pin 142 is moved downward in a direction indicated in the figure by an arrow. Disposing pin body 142A, in particular, of disposing pin 142 passes through the hollow section inside collecting pin body 141A. Fig. 22 corresponds to the state shown in Figs. 10 and 11, for example. Disposing pin body 142A moved downward comes into contact with portion 4E of the gel sheet collected with collecting pin body 141A and presses the portion downward. As a result, portion 4E of the gel sheet is pushed out of collecting pin body 141A and disposed outside collecting pin 141. Specifically, portion 4E of the gel sheet is collected inside container 9A (on a bottom surface thereof). The bottom surface of container 9A may be a dry surface without a high viscosity solution or the like applied thereto.

Collecting pin body 141A penetrating gel plate 6 breaks through both gel sheet 4 and film 2. However, as shown in Fig. 21, it is preferable that gel sheet 4 be alone accommodated in a hollow section 7C and film 2 be not accommodated therein. As a result, as shown in Fig. 22, it is preferable that portion 4E of the gel sheet be alone recovered in container 9A and film 2 be not recovered in container 9A.

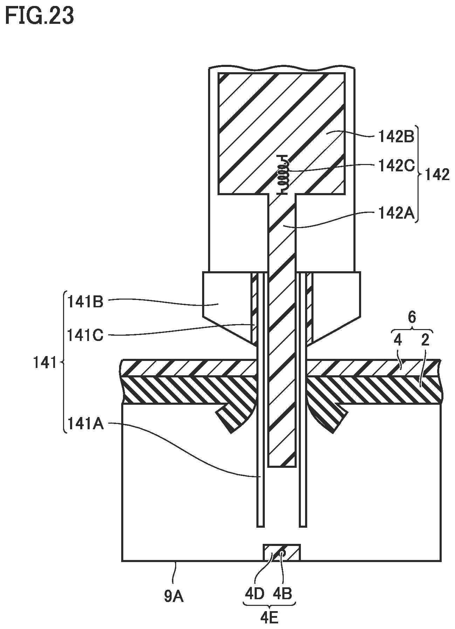

Fig. 23 is a schematic enlarged cross section for illustrating an eighth step of the collecting and disposing method according to the present embodiment. Referring to Fig. 23, after portion 4E of the gel sheet is placed on the bottom surface of container 9A, disposing pin body 142A ascends again. Fig. 23 corresponds to the states shown in Figs. 12 and 13, for example.

### (Materials)

Gel 4D is a gel obtained by gelatinizing gel material 4A having biocompatibility. Having biocompatibility as referred to herein means not exhibiting toxicity such as inflammation even when a biological tissue such as a cell comes into contact, and having a nature with low invasiveness to the cell. This can suppress damage attributed to the gel to the cell (or sample 4B). Gel material 4A is preferably transparent or semitransparent from the viewpoint of enabling observation of the position of sample 4B embedded. From the viewpoint of satisfying the above, gel material 4A is any selected from the group consisting of collagen, gelatin, fibrin, sodium alginate, gellan gum, agarose, hyaluronic acid, chitin, chitosan, polyethylene glycol, polyvinyl alcohol, and 2-methacryloyloxyethyl phosphorylcholine (MPC polymer).

Sample 4B as a target may for example be an isolated cell or an aggregate of cells. Herein, being isolated means both including only a single sample 4B in portion 4E of the gel sheet, as shown in Figs. 21 to 23, and including a plurality of samples 4B one by one such that they are spaced from one another, as indicated in a left half region of gel material 4A shown in Figs. 14 and 15. In contrast, as shown in a right half region of gel material 4A shown in Figs. 14 and 15, a mass of a plurality of samples 4B gathered is referred to as an aggregate. A cell as sample 4B may be any selected from the group consisting of myocardial cells, stem cells, nerve cells, and fibroblasts as primary cultured cells, established cell lines, and stem cell-derived differentiated cells.

For example, when a plurality of samples 4B are gathered in gel sheet 4 and form an aggregate, and the aggregate has a large size, in particular, collecting pin body 141A may puncture sample 4B and thus damage sample 4B. If sample 4B is damaged, it may be difficult to analyze a cross section of a tissue thereof, for example. Therefore, it is preferable to disperse the plurality of samples 4B to separate them from one another, as discussed above. This can suppress damage caused by puncture to sample 4B. Note that an aggregate may be formed, however, when the aggregate is small in size and can pass through the hollow section of collecting pin body 141A and a possibility of sample 4B being punctured by collecting pin body 141A is small.

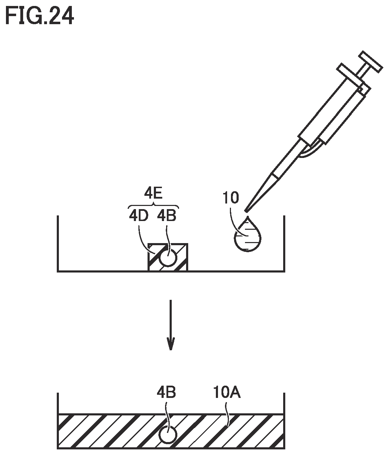

Fig. 24 is a schematic cross section for illustrating an example of a step of dissolving a gel from a portion of a gel sheet to recover a cell alone. Referring to Fig. 24, it is also possible to recover a cell (sample 4B) alone by dissolving gel 4D that supports and fixes sample 4B from portion 4E of the gel sheet cut out. Specifically, portion 4E of the gel sheet in which 5 x 10⁵ cells/mL of a fibroblast are disposed in gel 4D as sample 4B is accommodated inside an implement. A gel dissolving agent 10 is supplied into the implement. Gel dissolving agent 10 dissolves gel 4D. Thus, as illustrated in a figure on the lower side of Fig. 24, gel dissolving agent 10 will contain gel 4D in a dissolved state and thus be held in the implement as a gel dissolving agent 10A. It is in the form of liquid and can be discarded to recover sample 4B alone. For example, when gel 4D uses sodium alginate, an alginic acid-degrading enzyme or an EDTA (ethylenediaminetetraacetic acid) solution is used as gel dissolving agent 10.

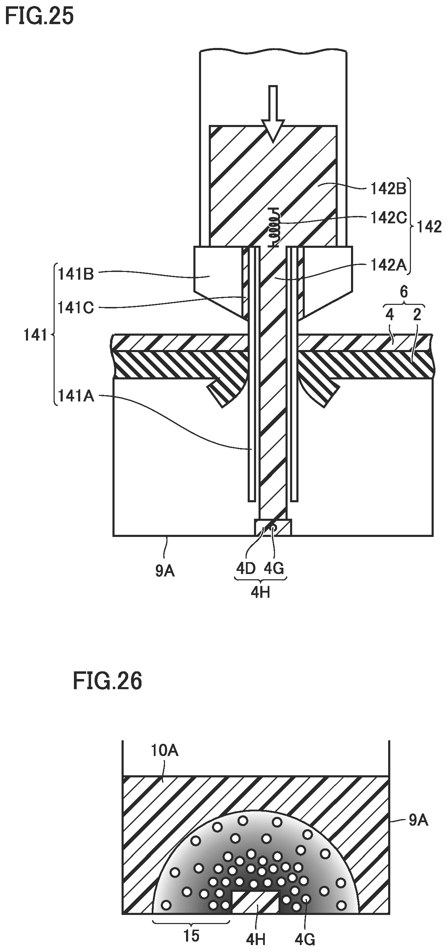

Fig. 25 is a schematic enlarged cross section in which the sample in FIG. 22 is replaced with a factor. Referring to Fig. 25, herein, a portion 4H of the gel sheet is shown instead of portion 4E of the gel sheet, and a factor 4G is shown instead of sample 4B. In the collecting and disposing method of Figs. 14 to 23, a target embedded in gel 4D may be any factor 4G selected from the group consisting of proteins, peptides, compounds, and particles. Accordingly, Figs. 14 to 17 also indicate a reference numeral 4G in parentheses. The proteins for factor 4G may be any selected from the group consisting of a fibroblast growth factor (FGF), a hepatocyte growth factor (HGF), a nerve growth factor (NGF), and a vascular endothelial growth factor (VEGF) as growth factors. Alternatively, the proteins for factor 4G may be glucosidase alpha as an enzyme. Furthermore, the proteins for factor 4G may be interleukin-2 (IL-2) or granulocyte colony-stimulating factor (G-CSF) as a cytokine. The peptides for factor 4G include either hormones or neuropeptides.

Factor 4G as the compounds may be any selected from the group consisting of a beta 1-adrenergic receptor agonist/antagonist, a Rho kinase inhibitor, and a Wnt signaling pathway related inhibitor. Factor 4G as the particles may be made of any selected from the group consisting of plastic, a lipid bilayer, and polysaccharides. These factor 4G proteins, peptides, and their compounds, as well as proteins, peptides, and their compounds adsorbed or contained in particles, can be slowly released, as will be described below.

Fig. 26 is a schematic enlarged cross section for illustrating a step of slowly releasing or eluting from the portion of the gel sheet shown in Fig. 25 a factor contained in the gel. Referring to Fig. 26, similarly as done in Fig. 24, a gel dissolving agent is supplied to a portion 4H of the gel sheet that is disposed in container 9A of Fig. 25 and has factor 4G embedded therein. The gel dissolving agent dissolves gel 4D and the gel is thus reserved in container 9A as gel dissolving agent 10A. In gel dissolving agent 10A, factor 4G contained in portion 4H of the gel sheet is slowly released from portion 4H of the gel sheet, and a concentration gradient 15 of factor 4G is created. A difference in concentration of factor 4G is caused between portion 4H of the gel sheet having factor 4G embedded therein and solution 10A free of factor 4G. The difference in concentration disperses factor 4G. Factor 4G contained in portion 4H of the gel sheet is slowly released from portion 4H of the gel sheet, and concentration gradient 15 of factor 4G is created. That is, after factor 4G is ejected from collecting pin body 141A, as shown in Fig. 25, factor 4G contained in portion 4H of the gel sheet can be slowly released, as shown in Fig. 26. While in Fig. 26 factor 4G may be slowly released as gel 4D is dissolved as described above, factor 4G may be eluted. Elution refers to elution of factor 4G contained in gel 4D even when gel 4D is not dissolved.

### (Function and Effect)

According to the present disclosure, collecting and disposing member 104 comprises collecting pin 141, disposing pin 142, and fixing holder 143. Collecting pin 141 has a hollowed shape and can cut out and collect a material to be treated. Collecting pin 141 is pressed against the material to cut out the material, and disposing pin 142 can be inserted into collecting pin 141 to come into contact with the cut out material inside collecting pin 141 and push the cut out material to dispose the cut out material outside collecting pin 141. Collecting pin 141 is fixed to fixing holder 143. The material to be treated is gel sheet 4 in which gel 4D that supports a target to be collected, or sample 4B, is formed into a sheet.

The material to be treated that is cut out with collecting pin 141 is pushed out by disposing pin 142 and thus disposed at a desired position (e.g., in container 9A or the like). This can suppress a problem concerned when pressure of liquid or the like is used to discharge from collecting pin 141 the material to be treated, such as collapse and scattering of a target such as sample 4B in the material to be treated. This allows the step of collecting sample 4B or the like to be performed with improved stably and the step of disposing sample 4B or the like to be performed with improved positional accuracy and reproducibility.

Collecting pin 141 is fixed to fixing holder 143. This can reduce a possibility of collecting pin 141 being unintentionally moved when the collecting pin receives an external vertical force. This allows the target to be cut out so as to have high stability and high reproducibility.

In collecting and disposing member 104, disposing pin 142 can pass through collecting pin 141 while being moved by first drive unit 144 in the upward/downward direction. Disposing pin 142 can thus eject portion 4E of the gel sheet accommodated in collecting pin 141 and dispose the portion at a desired position.

In collecting and disposing member 104, first drive unit 144 includes first motor 144A, rotatable disk member 144B attached to first motor 144A, and link member 144C extending in the upward/downward direction and having a first end fixed to a portion of an outer circumference of disk member 144B. Link member 144C in a direction in which it extends has a second end opposite to the first end, and as first motor 144A rotates disk member 144B, the second end moves in the upward/downward direction and hence moves disposing pin 142 in the upward/downward direction. First drive unit 144 converts rotation of first motor 144A and disk member 144B into upward/downward reciprocation via link member 144C. This allows disposing pin 142 to pass through a hollow section of collecting pin 141 and dispose portion 4E of the gel sheet at a desired position.

Collecting and disposing member 104 further comprises disposing pin holder fixing unit 145 provided between the second end and disposing pin 142 and fixed to disposing pin 142. Disposing pin holder fixing unit 145 allows disposing pin 142 to be stably fixed to disposing pin holder fixing unit 145.

In collecting and disposing member 104, disposing pin 142 includes disposing pin body 142A and disposing pin holder 142B that holds disposing pin body 142A. Spring 142C is installed in a region of disposing pin holder 142B adjacent to disposing pin body 142A. This allows disposing pin body 142A to effectively stably dispose portion 4E of the gel sheet or the like at a desired position.

Collecting and disposing member 104 further comprises second drive unit 146 capable of moving fixing holder 143 in the upward/downward direction. Second drive unit 146 includes second motor 146B, vertically extending screw 146C extending in the upward/downward direction and having one end (e.g., an upper end) with second motor 146B attached thereto, and screw coupling unit 146D that connects vertically extending screw 146C and fixing holder 143 together and is slidable in a direction in which vertically extending screw 146C extends. Second drive unit 146 converts rotation of second motor 146B and vertically extending screw 146C into upward/downward reciprocation via screw coupling unit 146D. This allows collecting and disposing mechanism 104A including collecting pin 141 to reciprocate as a whole in the upward/downward direction and thus cut a desired portion of gel sheet 4 with high positional accuracy.

According to the present disclosure, collecting and disposing apparatus 100 comprises collecting and disposing member 104 as described above, and a holding table (XY sample stage 101) that holds a material cut with collecting pin 141 to be treated. Collecting and disposing apparatus 100 thus configured has a function and effect of collecting and disposing member 104 as described above.

According to the present disclosure, in a collecting and disposing method, collecting and disposing member 104 as described above is prepared. A material to be treated is disposed under collecting pin 141, and collecting pin 141 is moved downward to cause its tip to cut the material and collects the cut material in collecting pin 141, and disposing pin 142 is moved downward to bring its tip into contact with and push the cut material in collecting pin 141 to dispose the cut material outside collecting pin 141.

When the tip of disposing pin 142 comes into contact with and pushes the material to be treated, the tip ejects the material to be treated. This can suppress a problem concerned when pressure of liquid or the like is used to discharge from collecting pin 141 the material to be treated, such as collapse and scattering of a target such as sample 4B in the material to be treated. This allows the step of collecting sample 4B or the like to be performed with improved stably and the step of disposing sample 4B or the like to be performed with improved positional accuracy and reproducibility.

In the presently disclosed collecting and disposing method, a gel supporting a target to be collected (i.e., sample 4B/factor 4G) is formed into a sheet on film 2 to provide gel sheet 4. Hollowed collecting pin 141 is used to puncture gel sheet 4 on film 2 to cut and accommodate a portion of gel sheet 4, or portion 4E/4H of the gel sheet, in collecting pin 141, and disposing pin 142 is used to press and thus push portion 4E/4H of the gel sheet out of collecting pin 141 to dispose the portion outside collecting pin 141. In the step of disposing outside collecting pin 141, disposing pin 142 is passed through collecting pin 141.

When disposing pin 142 presses portion 4E/4H of the gel sheet, the disposing pin pushes and thus ejects portion 4E/4H of the gel sheet out of collecting pin 141. This can suppress a problem concerned when pressure of liquid or the like is used to discharge portion 4E/4H of the gel sheet from collecting pin 141, such as collapse and scattering of a target such as sample 4B. This can improve stability in the step of collecting the target and improve positional accuracy and reproducibility in the step of disposing the target. Collecting and disposing apparatus 100 allowing the present collecting and disposing method to be applied thereto may comprise fixing holder 143 to which collecting pin 141 is fixed.

In the step of disposing outside collecting pin 141, after collecting pin 141 cuts out portion 4E/4H of the gel sheet to include the target, collecting pin 141 penetrates film 2 while holding portion 4E/4H of the gel sheet therein. The above-described method allows portion 4E/4H, as desired, of the gel sheet to be alone held inside collecting pin 141 without film 2 mixed and thus present together, and to be disposed at a desired position such as in container 9A.

### (Variations)

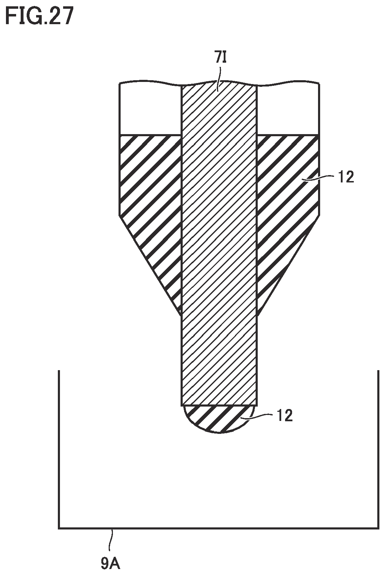

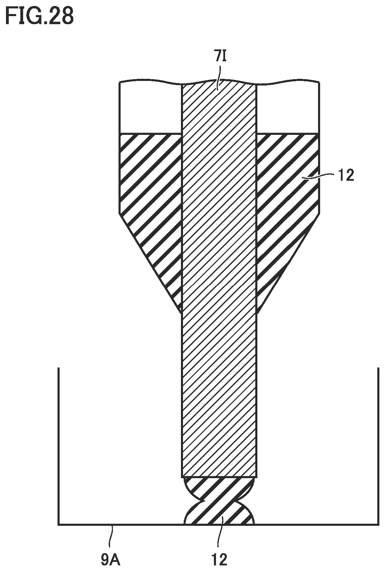

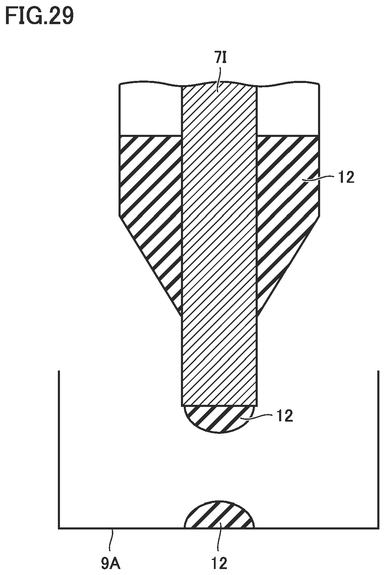

Fig. 27 is a schematic enlarged cross section for illustrating a first step of supplying a high viscosity solution into a container according to an exemplary variation. Fig. 28 is a schematic enlarged cross section for illustrating a second step of supplying the high viscosity solution into the container according to the exemplary variation. Fig. 29 is a schematic enlarged cross section for illustrating a third step of supplying the high viscosity solution into the container according to the exemplary variation. Referring to Figs. 27 to 29, an application needle 7I of a generally known application mechanism is used to supply container 9A (e.g., on a bottom surface thereof) with a high viscosity solution 12. Application needle 7I may be solid or hollow. Furthermore, a droplet dropping mechanism of a generally known dispenser may be used to drop high viscosity solution 12 onto the bottom surface of container 9A from above in the Z direction, as well as in Figs. 27 to 29.

As high viscosity solution 12, it is preferable to use cellulose, cellulose nanofiber, methylcellulose, carboxymethylcellulose, hydroxybutylcellulose, sodium alginate, sodium hyaluronate, or polyethylene glycol, for example.

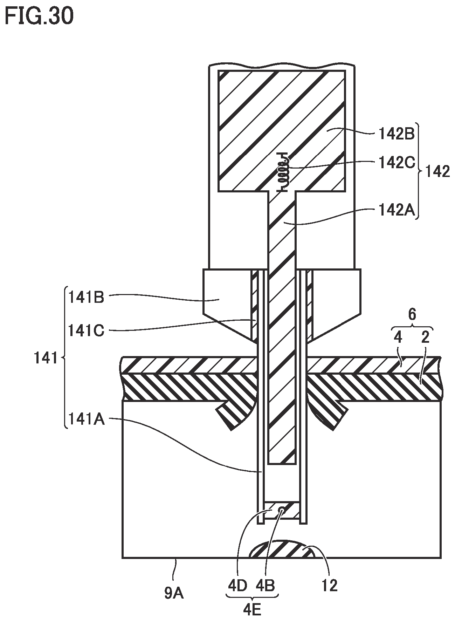

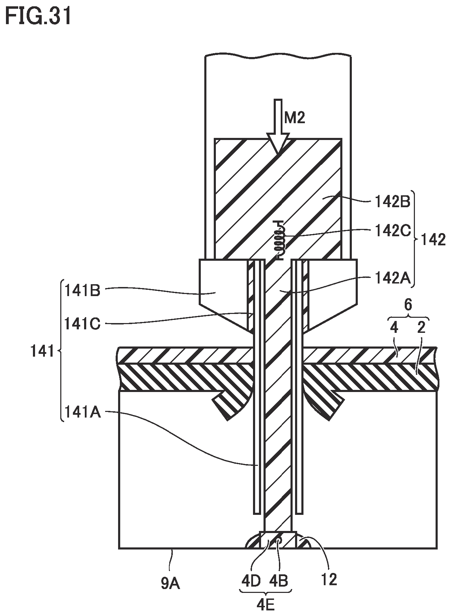

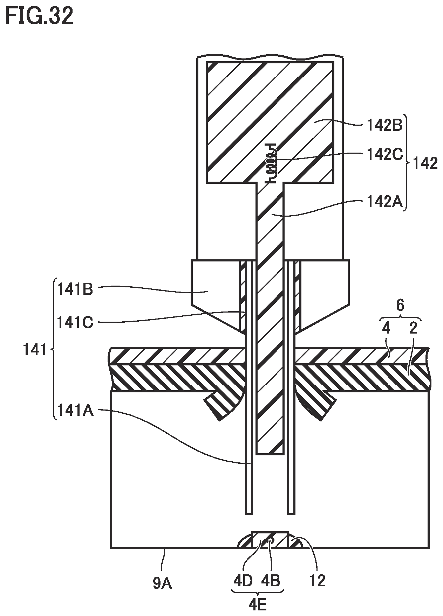

Fig. 30 is a schematic enlarged cross section corresponding to Fig. 21 in an exemplary variation in which a high viscosity solution is supplied. Fig. 31 is a schematic enlarged cross section corresponding to Fig. 22 in the exemplary variation in which the high viscosity solution is supplied. Fig. 32 is a schematic enlarged cross section corresponding to Fig. 23 in the exemplary variation in which the high viscosity solution is supplied. Referring to Figs. 30 to 32, collecting and disposing member 104 disposes portion 4E of a gel sheet, as done in Figs. 21 to 23, on high viscosity solution 12 supplied into container 9A. This allows portion 4E of the gel sheet to be fixed more firmly than when the portion is disposed on a dry surface of container 9A as shown in Figs. 21 to 23.

In addition, although not shown, portion 4E of the gel sheet collected with collecting pin 141 can also be supplied into liquid filling container 9A. In that case, while collecting pin 141 has portion 4E of the gel sheet accommodated therein, collecting pin 141 has its tip side with at least a partial region thereof immersed in that liquid and adjusted to a position at which portion 4E of the gel sheet is desired to be ejected. In this state, disposing pin 142 presses and thus ejects portion 4E of the gel sheet into the liquid. Adjusting the liquid in viscosity allows portion 4E of the gel sheet to be fixed in container 9A or the like.

Furthermore, in the present embodiment, sample 4B may be the cell described above. However, instead of the cell, sample 4B may be a biocompatible material in the form of a granule. As referred to herein, a biocompatible material is, for example, gel material 4A as has been described above. Gel material 4A may be a biomaterial. In that case, it will be gel material 4A granulated and floating in gel 4D provided in the form of a sheet. Gel 4D in the form of a sheet cut out of gel sheet 4 of the present embodiment is also applicable to an evaluation chip for drug response evaluation. More specifically, gel 4D in the form of the sheet cut out of gel sheet 4 is applicable to an evaluation chip for drug discovery and development, in particular.

### [Example 1]

Collecting and disposing apparatus 100 described in the above embodiment was used to collect and dispose a material to be treated. As the material to be treated, 5% agarose gel was formed into a sheet with no sample embedded therein to provide gel sheet 4 which was in turn disposed on film 2, as shown in Fig. 17, to provide gel plate 6. Film 2 was made of polyvinylidene chloride and had a thickness of about 10 µm.

Subsequently, as shown in Fig. 21, collecting pin 141 was moved from above downward substantially vertically to penetrate gel sheet 4 and film 2 to collect portion 4E of the gel sheet. This was done by using two types of collecting and disposing member 104 including collecting pin body 141A having an inner diameter of 0.7 mm and an outer diameter of 1 mm (hereinafter referred to as a first collecting pin) and collecting and disposing member 104 including collecting pin body 141A having an inner diameter of 1.1 mm and an outer diameter of 1.5 mm (hereinafter referred to as a second collecting pin). Herein, an inner diameter and an outer diameter are diameters.

Subsequently, as shown in Fig. 22, disposing pin body 142A having an outer diameter of 0.5 mm was inserted from above into a hollow section of each of the two types of collecting pins 141. The tip of disposing pin body 142A pushed out portion 4E of the gel sheet in collecting pin 141. Portion 4E of the gel sheet pushed out was disposed on a slide glass, and observed in morphology with a phase contrast microscope.

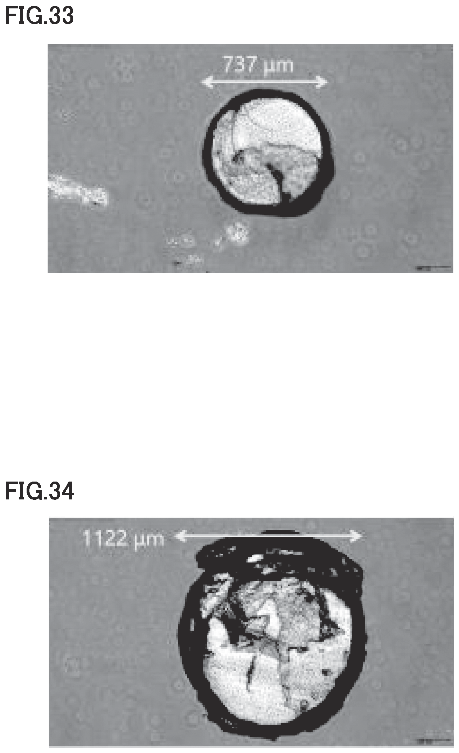

Fig. 33 is a photograph of a portion of a gel sheet collected with the first collecting pin. Fig. 34 is a photograph of a portion of a gel sheet collected with the second collecting pin. Referring to Figs. 33 and 34, the portions of the gel sheets each have a round shape as the hollow section of collecting pin 141 does. The portion of the gel sheet collected with the first collecting pin had a size (or diameter) of 737 µm, and the portion of the gel sheet collected with the second collecting pin had a size (or diameter) of 1122 µm.

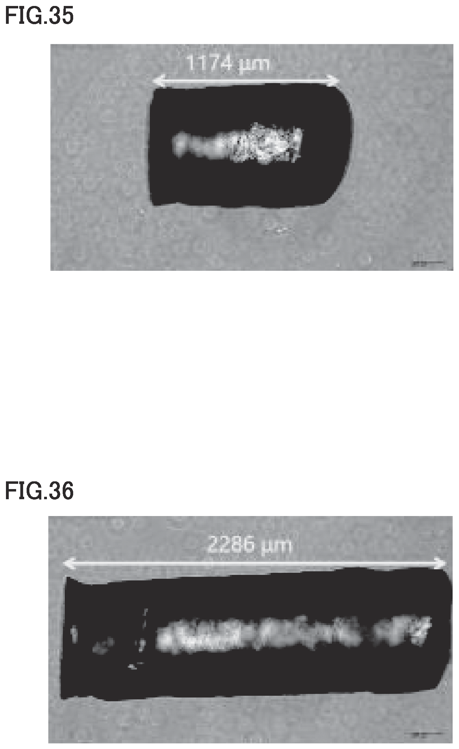

A test similar to the above was conducted for two types of gel sheets 4 different in thickness (i.e., a first gel sheet small in thickness and a second gel sheet larger in thickness than the first gel sheet). Fig. 35 is a photograph of a portion collected from the first gel sheet. Fig. 36 is a photograph of a portion collected from the second gel sheet. Referring to Figs. 35 and 36, the portion collected from the first gel sheet, which had a thickness of about 1000 µm, had a thickness of about 1174 µm, and the portion collected from the second gel sheet, which had a thickness of about 2000 µm, had a thickness of about 2286 µm. Thus, a portion collected from a gel sheet had a thickness depending on the thickness of the gel sheet.

From the above results, in collecting a gel sheet, controlling collecting pin body 141A in dimension and the gel sheet in thickness allows the gel sheet to be collected to have any shape and any size.

The inner diameter of the hollow section of collecting pin body 141A and the outer diameter (or the diameter) of disposing pin body 142A has a relationship, as follows. When collecting pin body 141A having an inner diameter of about 0.7 mm was used to collect a portion of a gel sheet and disposing pin body 142A having an outer diameter of 0.5 mm was used to push out the portion of the gel sheet, the portion of the gel sheet was stably pushed out. In contrast, when collecting pin body 141A having an inner diameter of about 1.1 mm was used to collect a portion of a gel sheet and disposing pin body 142A having an outer diameter of 0.5 mm was used to push out the portion of the gel sheet, it was difficult to stably push out the portion of the gel sheet. Therefore, in order to stably push out the gel sheet, it is preferable that the outer diameter of disposing pin body 142A be smaller than the inner diameter of collecting pin body 141A by about 0.2 mm.

The features of the embodiments (examples) described above may be applied in combination as appropriate in a range without contradiction in the art.

The embodiments disclosed herein should be considered as illustrative and not limiting in any respect. The scope of the present invention is defined by the terms of the claims, rather than the above description, and intended to encompass any modification equivalent in meaning and scope to the terms of the claims.

Hereinafter, a variety of manners of the present disclosure will be summarized as additional notes.

### (Additional Note 1)

A collecting and disposing member comprising:
a collecting pin having a hollowed shape and capable of cutting and collecting a material to be treated;
a disposing pin insertable into the collecting pin to come into contact in the collecting pin with the material cut out by the collecting pin as the collecting pin presses the material, and push the cut material to dispose the cut material outside the collecting pin; and
a fixing holder to which the collecting pin is fixed,
the material to be treated being a gel sheet, which is a gel formed into a sheet and supporting a target to be collected.

### (Additional Note 2)

The collecting and disposing member according to additional note 1, wherein the disposing pin can pass through the collecting pin while being moved by a first drive unit in an upward/downward direction.

### (Additional Note 3)

The collecting and disposing member according to additional note 2, wherein
the first drive unit includes a first motor, a rotatable disk member attached to the first motor, and a link member extending in the upward/downward direction and having a first end fixed to a portion of an outer circumference of the disk member, and
the link member in a direction in which it extends has a second end opposite to the first end, and as the first motor rotates the disk member, the second end moves in the upward/downward direction and hence moves the disposing pin in the upward/downward direction.

### (Additional Note 4)

The collecting and disposing member according to additional note 3, further comprising a disposing pin holder fixing unit provided between the second end and the disposing pin and fixed to the disposing pin.

### (Additional Note 5)

The collecting and disposing member according to any one of additional notes 1 to 4, wherein
the disposing pin includes a disposing pin body and a disposing pin holder that holds the disposing pin body, and
a spring is installed in a region of the disposing pin holder adjacent to the disposing pin body.

### (Additional Note 6)

The collecting and disposing member according to any one of additional notes 1 to 5, further comprising a second drive unit capable of moving the fixing holder in an upward/downward direction, wherein
the second drive unit includes a second motor, a vertically extending screw extending in the upward/downward direction and having one end with the second motor attached thereto, and a screw coupling unit that connects the vertically extending screw and the fixing holder together and is slidable in a direction in which the vertically extending screw extends.

### (Additional Note 7)

A collecting and disposing apparatus comprising:
the collecting and disposing member according to any one of additional notes 1 to 6; and
a holding table that holds a material to be cut out with the collecting pin to be treated.

### (Additional Note 8)

A collecting and disposing method comprising the steps of:
preparing the collecting and disposing member according to any one of additional notes 1 to 7; and
moving the collecting pin downward to cause a tip thereof to cut the material to be treated while the material is disposed under the collecting pin and collecting the cut material in the collecting pin, and moving the disposing pin downward to bring a tip thereof into contact with and push the cut material in the collecting pin to dispose the cut material outside the collecting pin.

### (Additional Note 9)

A collecting and disposing method comprising the steps of:
forming a gel into a sheet on a film to provide a gel sheet, the gel supporting a target to be collected; and
puncturing the gel sheet on the film with a hollow collecting pin to cut and collect a portion of the gel sheet in the collecting pin, and pressing the portion of the gel sheet with a disposing pin to push the portion of the gel sheet out of the collecting pin to dispose the portion of the gel sheet outside the collecting pin,
the step of puncturing including passing the disposing pin through the collecting pin.

### (Additional Note 10)

The collecting and disposing method according to additional note 9, wherein the step of puncturing includes causing the collecting pin to cut out the portion of the gel sheet to include the target, and subsequently causing the collecting pin to penetrate the film while the collecting pin holds the portion of the gel sheet therein.

### (Additional Note 11)

The collecting and disposing method according to additional note 9 or 10, wherein the target is embedded in the gel.

### (Additional Note 12)

The collecting and disposing method according to any one of additional notes 9 to 11, wherein the gel is a biocompatible gel material gelatinized.

### (Additional Note 13)

The collecting and disposing method according to any one of additional notes 9 to 12, wherein the target is an isolated cell or an aggregate of cells.

### (Additional Note 14)

The collecting and disposing method according to additional note 13, further comprising dissolving the gel to recover the target.

### (Additional Note 15)

The collecting and disposing method according to any one of additional notes 9 to 12, wherein
the target is any factor selected from the group consisting of a protein as a growth factor, a protein as an enzyme, a peptide, a compound, and a particle,
the compound is any selected from the group consisting of a beta 1-adrenergic receptor agonist/antagonist, a Rho kinase inhibitor, and a Wnt signaling pathway related inhibitor, and
the particle is any selected from the group consisting of plastic, a lipid bilayer, and polysaccharides.

### (Additional Note 16)

The collecting and disposing method according to additional note 15, wherein the factor is slowly released after the factor is ejected from the collecting pin.

### REFERENCE SIGNS LIST

1, 5 slide glass, 2 film, 3 spacer, 3A through hole, 4 gel sheet, 4A gel material, 4B sample, 4C fluid sample, 4D gel, 4E, 4H portion of gel sheet, 4G factor, 6 gel plate, 7I application needle, 8 plate, 9A container, 10, 10A gel dissolving agent, 12 high viscosity solution, 15 concentration gradient, 21 sheet fixing member, 24 collecting needle unit, 100 collecting and disposing apparatus, 101 XY sample stage, 101A stage penetrating section, 102 XY container stage, 104 collecting and disposing member, 104A collecting and disposing mechanism, 106 optical observation system, 141 collecting pin, 141A collecting pin body, 141B collecting pin holder, 142 disposing pin, 142A disposing pin body, 142B disposing pin holder, 142C spring, 143 fixing holder, 143f, 146f front surface, 143b back surface, 144 first drive unit, 144A first motor, 144B disk member, 144C link member, 144D1, 144D2 joint, 145 disposing pin holder fixing unit, 145a first portion, 145b second portion, 146 second drive unit, 146A movable holder, 146B second motor, 146C vertically extending screw, 146D screw coupling unit, 146E slide groove, FXD fixing portion.

## Claims

1. A collecting and disposing member comprising:
a collecting pin having a hollowed shape and capable of cutting and collecting a material to be treated;
a disposing pin insertable into the collecting pin to come into contact in the collecting pin with the material cut out by the collecting pin as the collecting pin presses the material, and push the cut material to dispose the cut material outside the collecting pin; and
a fixing holder to which the collecting pin is fixed,
the material to be treated being a gel sheet, which is a gel formed into a sheet and supporting a target to be collected.

2. The collecting and disposing member according to claim 1, wherein the disposing pin can pass through the collecting pin while being moved by a first drive unit in an upward/downward direction.

3. The collecting and disposing member according to claim 2, wherein
the first drive unit includes a first motor, a rotatable disk member attached to the first motor, and a link member extending in the upward/downward direction and having a first end fixed to a portion of an outer circumference of the disk member, and
the link member in a direction in which it extends has a second end opposite to the first end, and as the first motor rotates the disk member, the second end moves in the upward/downward direction and hence moves the disposing pin in the upward/downward direction.

4. The collecting and disposing member according to claim 3, further comprising a disposing pin holder fixing unit provided between the second end and the disposing pin and fixed to the disposing pin.

5. The collecting and disposing member according to claim 1 or 2, wherein
the disposing pin includes a disposing pin body and a disposing pin holder that holds the disposing pin body, and
a spring is installed in a region of the disposing pin holder adjacent to the disposing pin body.

6. The collecting and disposing member according to claim 1 or 2, further comprising a second drive unit capable of moving the fixing holder in an upward/downward direction, wherein
the second drive unit includes a second motor, a vertically extending screw extending in the upward/downward direction and having one end with the second motor attached thereto, and a screw coupling unit that connects the vertically extending screw and the fixing holder together and is slidable in a direction in which the vertically extending screw extends.

7. A collecting and disposing apparatus comprising:
the collecting and disposing member according to claim 1; and
a holding table that holds a material to be cut out with the collecting pin to be treated.

8. A collecting and disposing method comprising the steps of:
preparing the collecting and disposing member according to claim 1 or 2; and
moving the collecting pin downward to cause a tip thereof to cut the material to be treated while the material is disposed under the collecting pin and collecting the cut material in the collecting pin, and moving the disposing pin downward to bring a tip thereof into contact with and push the cut material in the collecting pin to dispose the cut material outside the collecting pin.

9. A collecting and disposing method comprising the steps of:
forming a gel into a sheet on a film to provide a gel sheet, the gel supporting a target to be collected; and
puncturing the gel sheet on the film with a hollow collecting pin to cut and collect a portion of the gel sheet in the collecting pin, and pressing the portion of the gel sheet with a disposing pin to push the portion of the gel sheet out of the collecting pin to dispose the portion of the gel sheet outside the collecting pin,
the step of puncturing including passing the disposing pin through the collecting pin.

10. The collecting and disposing method according to claim 9, wherein the step of puncturing includes causing the collecting pin to cut out the portion of the gel sheet to include the target, and subsequently causing the collecting pin to penetrate the film while the collecting pin holds the portion of the gel sheet therein.

11. The collecting and disposing method according to claim 9 or 10, wherein the target is embedded in the gel.

12. The collecting and disposing method according to claim 9 or 10, wherein the gel is a biocompatible gel material gelatinized.

13. The collecting and disposing method according to claim 9 or 10, wherein the target is an isolated cell or an aggregate of cells.

14. The collecting and disposing method according to claim 13, further comprising dissolving the gel to recover the target.

15. The collecting and disposing method according to claim 9 or 10, wherein
the target is any factor selected from the group consisting of a protein as a growth factor, a protein as an enzyme, a peptide, a compound, and a particle,
the compound is any selected from the group consisting of a beta 1-adrenergic receptor agonist/antagonist, a Rho kinase inhibitor, and a Wnt signaling pathway related inhibitor, and
the particle is any selected from the group consisting of plastic, a lipid bilayer, and polysaccharides.

16. The collecting and disposing method according to claim 15, wherein the factor is slowly released after the factor is ejected from the collecting pin.
